(19) Europäisches Patentamt / European Patent Office / Office européen des brevets

(11) **EP 4 667 938 A1**

(12) **EUROPEAN PATENT APPLICATION**

(43) Date of publication:
**24.12.2025 Bulletin 2025/52**

(21) Application number: **24305967.2**

(22) Date of filing: **19.06.2024**

(51) International Patent Classification (IPC):
*G01N 33/68* $^{(2006.01)}$

(52) Cooperative Patent Classification (CPC):
**G01N 33/6893;** G01N 2333/475; G01N 2333/515;
G01N 2333/96494; G01N 2800/245; G01N 2800/50

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Institut National de la Santé et de la Recherche Médicale**
  **75013 Paris (FR)**
• **Nantes Université**
  **44035 Nantes Cedex 1 (FR)**

(72) Inventors:
• **MASSET, Christophe**
  **44600 Saint Nazaire (FR)**
• **DANTAL, Jacques**
  **44360 Vigneux-de-Bretagne (FR)**
• **DEGAUQUE, Nicolas**
  **44300 Nantes (FR)**
• **ROUSSEAU, Olivia**
  **44340 Bouguenais (FR)**

(74) Representative: **Ipsilon**
**12 Avenue d'Italie**
**75013 Paris (FR)**

(54) **METHODS FOR PREDICTING THE RISK OF PANCREATIC TRANSPLANT FAILURE**

(57) The present disclosure relates to an *in vitro* method for predicting the risk of occurrence of a pancreatic transplant failure in a recipient intended to receive a pancreatic transplant, wherein the pancreatic transplant originates from a donor, the method comprising at least the steps of; a) providing a biological sample previously obtained from the recipient; b) determining a biological score from the level of at least one protein biomarker selected from matrix metalloproteinase 9 (MMP-9), Vascular endothelial growth factor (VEGF) and Angiopoietin-2 (ANGPT2), measured in the biological sample; c) determining a clinical score from a plurality of clinical parameters from the donor and the recipient, those parameters being associated with a pancreatic transplant failure; d) determining a predictive risk score from the combination of the biological score determined in step (b) and the clinical score determined in step (c); e) comparing the predictive risk score obtained at step (d) with a predetermined reference value, and; f) concluding that the recipient is at high risk of having a pancreatic transplant failure if the predictive risk score is higher than the predetermined reference value or concluding that the recipient is at low risk of having a pancreatic transplant failure if the predictive risk score is lower than the predetermined reference value.

EP 4 667 938 A1

**Description**

## FIELD OF THE DISCLOSURE

The present disclosure relates to the field of organ transplant. In particular, the present disclosure relates to methods for predicting or diagnosing the risk of occurrence of a pancreatic transplant failure, in particular an early pancreatic transplant failure. The present disclosure also relates to methods for predicting the risk of occurrence or for preventing a thrombosis post transplantation.

## BACKGROUND

The transplantation of a pancreas represents a highly effective treatment option for patients with, for instance, type 1 diabetes. Pancreas transplantation offers the potential for complete diabetes remission and cessation of insulin dependence. This surgery procedure, particularly when combined with a kidney transplant, has demonstrated significant benefits in terms of patient survival and renal graft longevity. However, despite these advantages, pancreas transplantation remains relatively uncommon. This is primarily due to its technical complexity and the high associated morbidity. Notably, 10 to 15% of pancreas transplants result in complete thrombosis of the pancreatic transplant within the first month, leading to an almost immediate transplant failure.

Various risk factors for early transplant failure have been identified in the literature. However, there is currently no sufficiently robust predictive scoring system to effectively guide clinicians in evaluating the benefice/risk for an individual considering a pancreas transplant. Presently, two scoring systems are primarily used in pancreas transplantation: the Pancreas Donor Risk Index (PDRI) and the Pre-Procurement Pancreas Allocation Suitability Score (P-PASS) (Axelrod, et al. Am. J. Transplant. 10, 837-845 (2010) or Finger, et al. Am. J. Transplant. 13, 1840-1849 (2013)).

PDRI is an American scoring system derived from the SRTR clinical registry, encompasses around ten clinical variables related to both donor and recipient factors. Despite its inception in 2010, its clinical applicability has been subject to debate, primarily due to the lack of external validation. The P-PASS, a European scoring system aimed at assessing the quality of pancreatic grafts for transplantation, is based on expert consensus but lacks construction and testing specifically for predicting graft survival.

Consequently, these existing scoring systems, while valuable, present notable limitations in accurately predicting the risk of pancreas graft failure post-transplantation.

The lack of reliable predictive methods represents a significant challenge in the field of pancreas transplantation. Without accurate risk assessment tools, healthcare providers face difficulties in optimizing patient outcomes and minimizing the risk of transplant failure.

Thus, there is an urgent need for a novel scoring system and methods that can reliably predict the risk of occurrence of pancreatic transplant failure, particularly in the early stages post-transplantation.

There is also a need for a novel scoring system and methods for predicting the risk of occurrence or for preventing thrombosis in a recipient intended to receive a pancreatic transplant.

There is also a need for novel solutions to guide clinicians in deciding whether to initiate a preventive treatment for allograft thrombosis in recipients.

There is also a need for novel solutions for the allocation of pancreatic grafts from a donor to a recipient, in particular in regard to the predicted risk of occurrence of a pancreatic transplant failure.

The present disclosure has for purpose to satisfy all or part of those needs.

## SUMMARY

The present disclosure relates to an *in vitro* method for predicting the risk of occurrence, or the likelihood of occurrence, of a pancreatic transplant failure in a recipient intended to receive a pancreatic transplant, wherein the pancreatic transplant originates from a donor, the method comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) determining a **clinical score** from a plurality of clinical parameters from the donor and the recipient, these clinical parameters being associated with a pancreatic transplant failure,
d) determining a **predictive risk score** from the combination of the biological score determined in step (b) and the clinical score determined in step (c),
e) comparing the predictive risk score obtained at step (d) with a predetermined reference value, and

f) concluding that the recipient is at high risk of having a pancreatic transplant failure if the predictive risk score is higher than the predetermined reference value or concluding that the recipient is at low risk of having a pancreatic transplant failure if the predictive risk score is lower than the predetermined reference value.

The proposed predictive risk score developed by the inventors has demonstrated its reliably to predict the risk of pancreatic transplant failure, particularly in the early stages, in particular about one month, post-transplantation. This predictive risk score has the advantage to be used in a method for predicting the benefit or risk or likelihood of occurrence of a pancreatic transplant failure in a recipient. The predictive risk score of the present disclosure has shown to be more accurate in predicting the risk of pancreatic graft failure in the first month post-transplant than the PDRI score, the current gold standard in this field (AUC = 0.74 vs 0.49 (PDRI)). The methods of the present disclosure aim to provide a robust predictive framework to assist clinicians in making informed decisions, ultimately improving patient care and transplant success rates.

The predictive risk score of the present disclosure has demonstrated consistent predictive ability in predicting pancreatic graft survival at one year post-transplant (AUC = 0.73), outperforming the PDRI score, which is currently the gold standard (AUC = 0.46). In addition to its predictive capacity, the identification of the three protein biomarkers (MMP-9, VEGF, and ANGPT2) in the recipient associated with pancreatic graft failure has the potential to inform new hypotheses about the pathophysiology of early pancreatic thrombosis.

In some embodiments, the biological sample at step (a) can be chosen from the group consisting of whole blood, plasma and serum. In some particular embodiments, the biological sample at step (a) is serum.

In some embodiments, the level of the at least one protein biomarker at step (b) can consist of the concentration of at least one protein biomarker expressed in terms of absolute or relative concentration. In some particular embodiments, the concentration of at least one protein biomarker can be expressed in terms of relative concentration.

In some embodiments, the biological score can be determined at step (b) using the formula (I): Formula (I) *: [log$_{10}$ (level of VEGF)] + [log$_{10}$ (level of ANGPT2)] - [log$_{10}$ (level of MMP-9)].*

In some embodiments, the level of the at least one biomarker at step (b) can be measured with Flow Cytometry, enzyme immunoassay or Mass Spectrometry.

In some embodiments, the plurality of clinical parameters as described herein can include the age of the donor, the Body Mass Index (BMI) of the donor, the cold ischemia time of the pancreatic transplant and/or the presence or absence of renal replacement treatment from the recipient.

In some particular embodiments, the plurality of clinical parameters as described herein can consists of the age of the donor, the Body Mass Index (BMI) of the donor, the cold ischemia time of the pancreatic transplant and the presence or absence of renal replacement treatment from the recipient.

The clinical score is determined or calculated to assess the risk or probability of pancreatic transplant failure, preferably the risk or probability of pancreatic transplant failure at one-month post-transplantation in a recipient.

In some embodiments, the clinical score as described herein can be determined for a recipient using a formula (II) :

$$\text{Formula (II):}$$

$$p(1|x) = \frac{\exp\left(\beta_0 + \sum_{i=1}^{p} \beta_i x_i\right)}{1 + \exp\left(\beta_0 + \sum_{i=1}^{p} \beta_i x_i\right)}$$

wherein:

- "$p(1|x)$" represents the clinical score,
- $x = (x_1, ..., x_i, ... , x_p)$ a vector of size p with the values of the clinical parameters,

- $\beta = \begin{pmatrix} \beta_1 \\ ... \\ \beta_p \end{pmatrix}$ a vector of coefficients determined with the multivariable logistic regression, and

- $\beta_0$ refers to the intercept coefficient of the multivariate logistic regression model.

In some embodiments, the predictive risk score can be determined using a formula (III) :

(Formula III) :

$$p(1|s_x) = \frac{\exp\left(\beta_0 + \beta_{PFS}s_{x,PFS} + \beta_{clinique}s_{x,clinique}\right)}{1 + \exp\left(\beta_0 + \beta_{PFS}s_{x,PFS} + \beta_{clinique}s_{x,clinique}\right)}$$

wherein:

- "$p(1|s_x)$" represents the predictive risk score for a recipient $x$,
- $s_x = (s_{x,PFS}, s_{x,clinique})$ a vector with

   $s_{X,PFS}$ represents the obtained biological score for the recipient $x$ as described herein,
   $s_{X,clinique}$ represents the obtained clinical score for the recipient $x$ as described herein,

- $\begin{pmatrix} \beta_{PFS} \\ \beta_{clinique} \end{pmatrix}$ corresponds to the coefficients of the multivariable logistic regression model, and
- $\beta_0$ represents the intercept coefficient of the multivariable logistic regression model.

In some embodiments, the predetermined reference value can consist of a value determined from a plurality of recipients having received a pancreatic transplant and for whom the occurrence or non-occurrence of a pancreatic transplant failure is known. In some particular embodiments, the occurrence or non-occurrence of a pancreatic transplant failure can be known early post-transplantation, in particular at about one-month post-transplantation.

In some embodiments, the risk of occurrence of a pancreatic transplant failure can consist of :

- determining the risk, or the likelihood, of occurrence of early pancreatic transplantation failure at one month or more in the recipient after having been transplanted with the pancreatic transplant; and/or
- determining the benefit/risk balance of establishing a treatment to prevent the occurrence of thrombosis.

In some embodiments, the recipient can have a diabetes, a chronic pancreatitis, a pancreatic cancer and/or a pancreatic insufficiency.

In some embodiments, the recipient and the donor consists of mammals, in particular the recipient and the donor are humans.

In some embodiments, the prediction of pancreatic transplant failure can encompass the risk of failures occurring at one month or more post transplantation.

The present disclosure also relates to the use of a **predictive risk score** for predicting the risk of a pancreatic transplant failure in a recipient intended to receive a pancreatic transplant, the predictive risk score being determined from the combination of a biological score and a clinical score; wherein

   the **biological score** is determined from the level of at least one protein biomarker selected from matrix metalloproteinase 9 (MMP-9), Vascular endothelial growth factor (VEGF) and Angiopoietin-2 (ANGPT2), measured in a biological sample previously obtained from the recipient, and
   the **clinical score** is determined from a plurality of clinical parameters from the donor and the recipient from whom the pancreatic transplant originated, the clinical parameters being associated with a pancreatic transplant failure.

The present disclosure also relates to an *in vitro* method for predicting the risk of occurrence of a pancreatic transplant failure in a recipient intended to receive a pancreatic transplant, comprising at least the steps of:

   a) providing a biological sample previously obtained from the recipient,
   b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
   c) comparing the biological score at step b) with a predetermined reference biological score determined in at least one reference recipient, wherein the reference recipient did not receive a pancreatic transplant; and, optionally,
   d) concluding that the recipient is at high risk of having a pancreatic transplant failure if the biological score at step b) is lower than the predetermined reference biological score.

The present disclosure also relates to the use of a **biological score** for predicting the risk of a pancreatic transplant failure in a recipient intended to receive a pancreatic transplant, wherein the **biological score** is determined from the level of at least one protein biomarker selected from matrix metalloproteinase 9 (MMP-9), Vascular endothelial growth factor (VEGF) and Angiopoietin-2 (ANGPT2), measured in a biological sample previously obtained from the recipient.

The present disclosure also relates to a method for preventing a thrombosis in a recipient intended to receive a pancreatic transplant, comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) determining a **clinical score** from a plurality of clinical parameters from the donor and the recipient, these parameters being associated with a pancreatic transplant failure,
d) determining a **predictive risk score** from the combination of the biological score determined in step (b) and the clinical score determined in step (c),
e) comparing the predictive risk score obtained at step (d) with a predetermined reference value, and
f) concluding that the recipient is at high risk of having a thrombosis if the predictive risk score is higher than the predetermined reference value.

The present disclosure also relates to a method for predicting the risk of occurrence of a thrombosis in a recipient intended to receive a pancreatic transplant, comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) determining a **clinical score** from a plurality of clinical parameters from the donor and the recipient, these parameters being associated with a pancreatic transplant failure,
d) determining a **predictive risk score** from the combination of the biological score determined in step (b) and the clinical score determined in step (c),
e) comparing the predictive risk score obtained at step (d) with a predetermined reference value, optionally,
f) concluding that the recipient is at high risk of having a thrombosis if the predictive risk score is higher than the predetermined reference value.

## BRIEF DESCRIPTION OF THE FIGURES

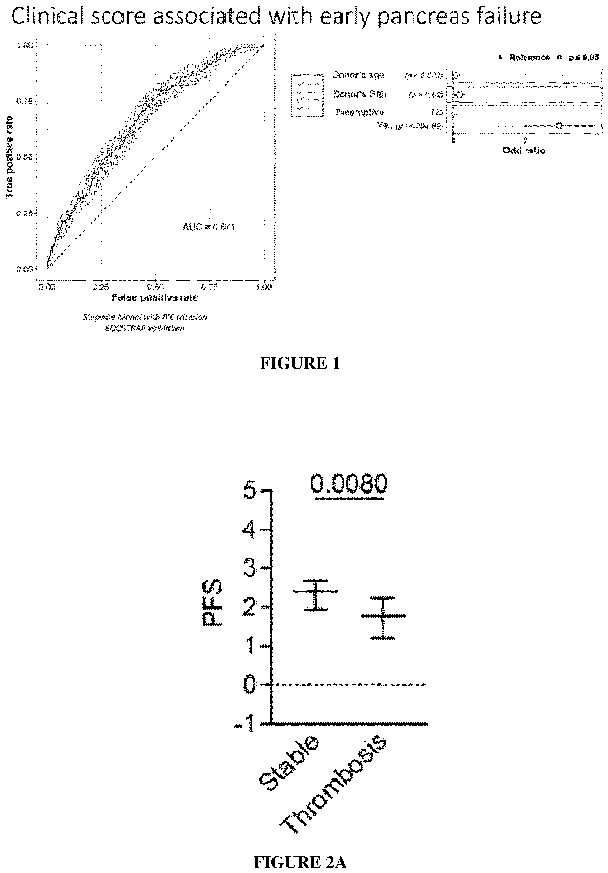

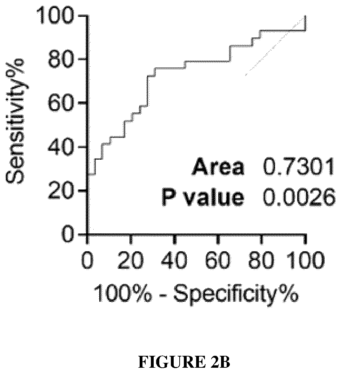

**Figure 1** shows a logistic regression curve measuring the accuracy of the three parameters determining the clinical score, *i.e.*, the age of the donor, the Body Mass Index (BMI) of the donor, and the preemptive status of the recipient (presence or absence of pre-transplant hemodialysis). The y axis (ordinate) represents the true positive rate (Sensitivity in percentage). The x axis (abscissa) represents the false positive rate (100 - Specificity in percentages). **Figure 2** represents the establishment of the biological score. **Figure 2A** shows a diagram of the distribution of values of the biological scores according to the group of individuals. Ordinate: Biological score in arbitrary units (AU). Abscissa: (from left to right) control group (stable) and case group with thrombosis. p= 0.008. **Figure 2B** shows a logistic regression curve measuring the accuracy of the biological score. Ordinate: Sensitivity in percentage. Abscissa: (from left to right) 100 - Sensitivity in percentage.

## DETAILED DESCRIPTION

### Definitions

The terms used in the present specification generally have their ordinary meanings in the art. Certain terms are discussed below, or elsewhere in the present disclosure, to provide additional guidance in describing the products and methods of the presently disclosed subject matter.

Exemplary methods and materials are described below, although methods and materials similar or equivalent to those described herein can also be used in the practice or testing of the present invention. Units, prefixes, and symbols are denoted in their International System of Units (SI) accepted form.

The following definitions apply in the context of the present disclosure.

As used in this specification and the appended claims, the singular forms "**a**", "**an**" and "**the**" include plural referents unless the content clearly dictates otherwise. For example, "a biomarker" is understood to represent one or more biomarker(s). As such, the terms "a" (or "an"), "one or more" and "at least one" can be used interchangeably herein.

Furthermore, the term "**and/or**", where used herein, is to be taken as specific disclosure of each of the two specified features or components with or without the other. Thus, the term "and/or" as used in a sentence such as "A and/or B" herein is intended to include "A and B", "A or B", "A" (alone), and "B" (alone).

Furthermore, the term "**optionally**", where used herein, is used to denote that an element or component place after the term optionally in the sentence is discretionary and may or may not be present within the described object or composition or combination. Thus, for instance, the term "optionally" as used in a sentence such as "A and, optionally B" herein is intended to denote that A is mandatory and B is discretionary and may or may not be present.

The term "**about**" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the art. Alternatively, "about" can mean a range of up to 20%, or up to 10%, or up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

Within the meaning of the invention, "**biomarker**" intends to refer to a biologically derived indicator (such as a metabolite) of a process, event, or condition (such as aging, disease). A biomarker is a quantifiable characteristic that is objectively measured and evaluated as an indicator of normal or pathogenic biological processes, or of pharmacologic responses to a therapeutic intervention. It can be any substance, structure, or process that can be measured in the body or its products and influence or predict the incidence of outcome or disease, the effect of a treatment, or an intervention. A biomarker can be a biological molecule, such as, for example, a nucleic acid, peptide, protein, hormone, and the like. In the present disclosure, matrix metalloproteinase 9 (MMP-9), Vascular endothelial growth factor (VEGF) and Angiopoietin-2 (ANGPT2) consist of biomarkers.

It is understood that aspects and embodiments of the present disclosure described herein include "**comprising**," "**consisting of**," and "**consisting essentially of**" aspects and embodiments. The word "comprise," or variations such as "comprises," or "comprising," will be understood to imply the inclusion of the stated element(s) but not the exclusion of any other elements. The term "consisting of" implies the inclusion of the stated element(s), to the exclusion of any additional elements. The term "consisting essentially of" implies the inclusion of the stated elements, and possibly other element(s) where the other element(s) do not materially affect the characteristic(s) of the stated elements. It is understood that the different embodiments of the disclosure using the term "comprising" or equivalents cover the embodiments where this term is replaced with "consisting of" or "consisting essentially of".

As used herein, the terms "**prevent**" or "**preventing**" (and grammatical variants thereof) with respect to a disease relate to prophylactic treatment of a disease, *e.g.*, in an individual at high risk of thrombosis, or at risk for developing thrombosis after a pancreatic transplantation. Prevention may include, but is not limited to, preventing, or delaying onset or progression of a disease and/or maintaining one or more symptoms of a disease at a desired or sub-pathological level. The term "prevent" does not require the 100% elimination of the possibility or likelihood of occurrence of the event. Rather, it denotes that the likelihood of the occurrence of the event has been reduced with the application of a method as described herein. More particular, "prevent" or "preventing" refers to a decrease in the risk of occurrence of a thrombosis or transplant failure in a recipient. As indicated above, the prevention may be complete, *i.e.*, no detectable symptoms or transplant failure, or partial, such that fewer symptoms or less severity of thrombosis are observed than would likely occur absent treatment.

Within the disclosure, the term "**significantly**" used with respect to a change intends to mean that the observed change is noticeable and/or it has a statistic meaning.

As used herein, the term "**recipient**" refers to an individual that is intended to receive a pancreas or a pancreatic tissue transplant from a donor. An individual can be mammal animal, in particular a human.

In the context of the present disclosure, the expression "**intended to receive**" means that a recipient is indented to undergo a pancreatic transplantation surgery.

As used herein, the term "**donor**" refers to an individual that provides a pancreas or a pancreatic tissue intended for transplantation into a recipient. An individual can be mammal animal, in particular a human.

As used herein, the expression "**post transplantation**" refers to the period that occur after the day of a transplant surgery.

The terms "**determine**" and "**measure**", or any equivalent thereof used within the present disclosure in relation with the biomarkers intend to mean the quantification of the biomarkers in a biological sample. The quantification is a measure of a quantity or level of a biomarker which may be expressed in volume, in mole, in weight, in weight by weight or by volume of the matrix containing the biomarker, such as a concentration, in particular a molar concentration. For example, a quantification of a biomarker may be expressed in ng/ml or pg/ml. The concentration of a biomarker may be expressed as an absolute concentration or expressed relatively to the quantification of another biomarker or to a reference (or standard). The determination of the level or quantity of a biomarker may be carried out by any known techniques in the art applicable to the concerned biomarker. In some embodiments, measured of a biomarker in a biological sample intends to mean the quantification of the given biomarker in the biological sample.

As used herein, the expression "**pancreatic transplant failure**" refers to the likelihood or probability that a transplanted pancreatic organ or tissue will not successfully achieve its intended biological function in the recipient. This is particularly relevant in the early stages post-transplantation, specifically within one month post transplantation. Typically, pancreatic transplant failure can result from the occurrence of thrombosis, and/or the failure to achieve persistent insulin independence, and/or undetectable C-peptide value, and/or necessitating surgical removal of the pancreas, and/or requiring subsequent transplantation of another pancreatic organ or tissue, and/or transplantation of islets of Langerhans.

The intercept coefficient of the model, i.e., $\beta_0$, is the expected value of $p(1|X)$ when all independent variables X are equal to zero.

As used herein, the term "**computer system**" refers to any and all devices capable of storing and processing information and/or capable of using the stored information to control the behavior or execution of the device itself, regardless of whether such devices are electronic, mechanical, logical, or virtual in nature. The term "computer system" can refer to a single computer, but also to a plurality of computers working together to perform the function described as being performed on or by a computer system. A method implemented using a computer system is referred to as a "computer-implemented method".

All lists of items are intended to and should be interpreted as Markush groups. Thus, all lists can be read and interpreted as items "selected from the group consisting of' ... list of items ... "and combinations and mixtures thereof."

In the description of the various embodiments of the present disclosure, various embodiments or individual features are disclosed. As will be apparent to the ordinarily skilled practitioner, all combinations of such embodiments and features are possible and can result in preferred executions of the present disclosure.

## Method and uses for predicting the risk of a pancreatic transplant failure

The present disclosure relates to an *in vitro* method for predicting the risk, or the likelihood, of occurrence of a pancreatic transplant failure in a recipient intended to receive a pancreatic transplant, wherein the pancreatic transplant originates from a donor, the method comprising at least the steps of:

   a) providing a biological sample previously obtained from the recipient,
   b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
   c) determining a **clinical score** from a plurality of clinical parameters from the donor and the recipient, these clinical parameters being associated with a pancreatic transplant failure,
   d) determining a **predictive risk score** from the combination of the biological score determined in step (b) and the clinical score determined in step (c),
   e) comparing the predictive risk score obtained at step (d) with a predetermined reference value, and, optionally,
   f) concluding that the recipient is at high risk of having a pancreatic transplant failure if the predictive risk score is higher than the predetermined reference value or concluding that the recipient is at low risk of having a pancreatic transplant failure if the predictive risk score is lower than the predetermined reference value.

The present disclosure also relates to the uses of a **predictive risk score** for predicting the risk of occurrence of a pancreatic transplant failure in a recipient intended to receive a pancreatic transplant, the predictive risk score being determined from the combination of a biological score and a clinical score;

   wherein the **biological score** is determined from the level of at least one protein biomarker selected from matrix metalloproteinase 9 (MMP-9), Vascular endothelial growth factor (VEGF) and Angiopoietin-2 (ANGPT2), measured in a biological sample previously obtained from the recipient, and
   the **clinical score** is determined from a plurality of clinical parameters from the donor and the recipient, the clinical parameters being associated with a pancreatic transplant failure.

The methods disclosed herein may be implemented at any time prior to a planned or urgent pancreas transplantation. Typically, these methods may be initiated by a clinician seeking to assess the probability or risk of occurrence of pancreatic transplant failure at an early stage. This risk assessment aids the clinician in making informed decisions regarding the suitability of proceeding with pancreatic transplantation, as well as determining the necessity of anti-thrombotic treatment.

In some embodiments, the methods and uses as described in the present disclosure are carried out *in vitro.*

In some embodiments, a pancreatic transplant obtained from a donor can encompass the entire pancreas organ or a tissue thereof.

In some embodiments, the methods and uses of the present disclosure can predict the risk, or the likelihood of occurrence, of an early pancreatic transplant failure. This prediction of the risk of an early pancreatic transplant failure can encompass transplant failures occurring at about one month or more post transplantation. One month period post transplantation can encompass from about 25 to 35 days post-transplantation.

In some particular embodiments, the prediction of the risk of pancreatic transplant failure can encompass transplant failures occurring at about 25 days, or about 26 days, or about 27 days, or about 28 days, or about 29 days, or about 30 days, or about 31 days, or about 32 days, or about 33 days, or about 35 days post-transplantation.

In some embodiments, the sequence for implementing step b) and step c) within the method is not fixed and can be interchanged. Therefore, step b) can be performed before, at the same time as, of after step c).

In some embodiments, the recipient in need thereof may have a disease or disorder necessitating a pancreatic transplantation surgery. In some embodiments, the recipient in need thereof may have a diabetes, a chronic pancreatitis, a pancreatic cancer and/or a pancreatic insufficiency.

In some embodiments, the recipient may have or suffer of a diabetes.

Diabetes can be a type 1 diabetes, type 2 diabetes, monogenic diabetes, secondary diabetes and/or latent autoimmune diabetes in adults (LADA).

In some particular embodiments, the recipient may have or suffer of a type 1 diabetes.

## Biological score

The *in vitro* method as described herein comprises a step of determining a **biological score** from the level of at least one protein biomarker selected from matrix metalloproteinase 9 (MMP-9), Vascular endothelial growth factor (VEGF) and Angiopoietin-2 (ANGPT2), measured in the biological sample.

This step of determining a biological score is performed after step a) of the method as described herein.

In some embodiments, this step of determining a **biological score** can be implement at step (b) of the method after, or as the same time as, or before, step c).

The level of one or more biomarker(s) is measured in a biological sample previously collected from a recipient to be tested. The recipient is generally the individual intended to receive a pancreatic transplant from a given donor.

In some embodiments, the biological sample, previously collected, can be selected from whole blood, plasma or serum.

In particular embodiments, the biological sample can be plasma.

Plasma is the liquid component of blood after cells are removed, while serum is the liquid remaining after blood has clotted and clotting factors have been removed.

In some embodiments, the biological sample at step (a) may be obtained from the recipient at any time prior to the planned or intended pancreas transplantation event. Typically, this is determined by the clinician for predicting the risk of pancreatic transplant failure of a recipient.

In some embodiments, the biological sample may be obtained from the recipient at various intervals before the planned or intended pancreas transplantation.

In some embodiments, the biological sample may be obtained from the recipient up to at least about two years before the planned or intended pancreas transplantation.

In some embodiments, the biological sample may be obtained from the recipient at least about one year, or nine months, or six months, or five months, or four months, or three months, or two months, or one month before the planned or intended pancreas transplantation.

The method may also be implemented in urgence cases. This implies thus that the biological sample may be obtained from the recipient a few weeks or hours before the planned or intended pancreas transplantation. Illustratively, the biological sample may be obtained from the recipient at least about one hour, or two hours, or six hours, or twelve hours or one day, or two days, or six days, or one week before the planned or intended pancreas transplantation.

In some embodiments, the biological sample at sept (a) can be previously obtained from the recipient at least one hour, or two hours, or six hours, or twelve hours or one day, or two days, or six days, or one week before the intended pancreas transplantation.

In some embodiments, the protein biomarkers are selected from matrix metalloproteinase 9 (MMP-9), Vascular endothelial growth factor (VEGF) and Angiopoietin-2 (ANGPT2).

In some embodiments, the biological score is determined from the level of matrix metalloproteinase 9 (MMP-9), Vascular endothelial growth factor (VEGF) and Angiopoietin-2 (ANGPT2).

Methods for measuring or quantifying the level or quantity of protein biomarkers in a biological sample are well known in the art. In some embodiments, the level of protein biomarkers, in particular MMP-9, VEGF and/or ANGPT2, can be quantified by a Flow Cytometry, an enzyme immunoassay or a Mass Spectrometry.

An enzyme immunoassay can be an Enzyme-linked immunosorbent assay (ELISA), an Enzyme multiplied immunoassay technique (EMIT), a Fluorescent enzyme immunoassays (FEIAs), a Chemiluminescent immunoassays (CLIAs), a Radioimmunoassays, or an automated immunoassay system (ELLA).

In some particular embodiments, the measure of the level or quantity of protein biomarkers, in particular MMP-9, VEGF and/or ANGPT2, in a biological sample can be quantified by ELISA or ELLA.

In some embodiments, the concentration of a biomarker may be expressed as an absolute concentration or expressed relatively to the quantification of another biomarker or to a reference (or standard). Illustratively, a reference can be a ubiquity protein.

The determination of the level or quantity of a biomarker may be carried out by any known techniques in the art applicable to the concerned biomarker.

In some embodiments, measured of a biomarker in a biological sample intends to mean the quantification of the given biomarker in the biological sample.

From the obtained level or quantity of the protein biomarkers, it is determined a biological score. The establishment of a biological score based on direct measurement of the three biomarkers facilitates the implementation of this prediction method in routine clinical practice, in comparison to the use of a principal component analysis.

In some embodiments, the biological score can be determined at step (b) using formula (I):

$$[\log_{10} (level\ of\ VEGF)] + [\log_{10} (level\ of\ ANGPT2)] - [\log_{10} (level\ of\ MMP\text{-}9)]. \qquad \text{Formula (I)}:$$

Log10, also known as common logarithm, is the logarithm to the base 10 that is well known in the art.

**Clinical score**

The *in vitro* method as described herein also comprises a step of determining a **clinical score** from a plurality of clinical parameters from the donor and the recipient.

In some embodiments, this step of determining a **clinical score** can be implement at step (c) of the method after, or as the same time as, or before step (b).

These plurality of clinical parameters associated with a pancreatic transplant failure are well described in the present domain. The clinical parameters of particular interest can be selected using biostatistical selection (stepwise logistic regression) from a list of clinical parameters associated with the risk factors described in the literature.

In some embodiments, the number of clinical parameters used for determining the clinical score can be between two to ten clinical parameters.

In some embodiments, the number of clinical parameters used for determining the clinical score can be between four to ten clinical parameters.

In some embodiments, the number of clinical parameters used for determining the clinical score can be of four or more clinical parameters.

In some embodiments, the clinical score is determined from at least four clinical parameters associated with a pancreatic transplant failure.

In some embodiments, the clinical score is determined from at least three clinical parameters of the donor associated with a pancreatic transplant failure, and at least one clinical parameter of the recipient associated with a pancreatic transplant failure.

Illustratively, the clinical parameters associated with a pancreatic transplant failure are described in Axelrod, et al. Am. J. Transplant. 10, 837-845 (2010) or Finger, et al. Am. J. Transplant. 13, 1840-1849 (2013), herein incorporated by reference.

In some embodiments, the clinical parameters associated with a pancreatic transplant failure can be chosen among the list of the parameters evaluated in the PDRI score.

In some embodiments, the clinical parameters associated with a pancreatic transplant failure may be chosen among, without limitation, the age of the donor, the Body Mass Index (BMI) of the donor, the height of the donor, the serum creatinine level of the donor, the gender of the donor, the ethnicity (African-American, Asian, Caucasian) of the donor, the presence or absence of a cerebral vascular accident (CVA) of the donor, the donation after cardiac death (DCD) of the donor, the transplantation type, the presence or absence of a renal replacement treatment of the recipient, and the cold ischemia time (CIT) of the pancreatic transplant from the donor.

In some embodiments, the clinical parameters associated with a pancreatic transplant failure may include at least the age of the donor, the Body Mass Index (BMI) of the donor, the cold ischemia time of the pancreatic transplant from the donor and the presence or absence of renal replacement treatment from the recipient.

Three, or two, or one further clinical parameter(s) of the donor may be included in the formula for determining the clinical score without significantly modifying the specificity of the obtained clinical score.

In some embodiments, a renal replacement treatment can be a dialysis (hemodialyse) and/or a kidney transplantation.

In some particular embodiments, the clinical parameters associated with a pancreatic transplant failure includes the age of the donor, the Body Mass Index (BMI) of the donor, the cold ischemia time of the pancreatic transplant from the donor and the presence or absence of renal replacement treatment from the recipient, and optionally three, or two or one further parameters among the height of the donor, the serum creatinine level of the donor, the gender of the donor, the ethnicity of the donor, the presence or absence of a cerebral vascular accident (CVA) of the donor, the donation after cardiac death (DCD) of the donor and/or the transplantation type.

In some embodiments, the clinical score of a recipient can be determined using the formula (II):

$$p(1|x) = \frac{\exp\left(\beta_0 + \sum_{i=1}^{p}\beta_i x_i\right)}{1 + \exp\left(\beta_0 + \sum_{i=1}^{p}\beta_i x_i\right)}$$

wherein:

- "$p(1|x)$" represents the clinical score,
- $x = (x_1, ..., x_i, ..., x_p)$ a vector of size $p$ with the values of the clinical parameters,

- $\beta = \begin{pmatrix} \beta_1 \\ ... \\ \beta_p \end{pmatrix}$ a vector of coefficients determined with the multivariable logistic regression, and

- $\beta_0$ refers to the intercept coefficient of the multivariate logistic regression model.

In some embodiments, p can consist of 1, 2, 3, 4, 5, 6, 7, 8, 9, or 10.

In some embodiments, $x_p$ can comprise $x_5$; $x_5$ representing a value of a a fifth clinical parameter.

In some embodiments, $x_p$ can comprise $x_5$ and $x_6$; $x_5$ representing a value of a fifth clinical parameter and $x_6$ representing a value of a sixth clinical parameter.

In some embodiments, $x_p$ can comprise $x_5$, $x_6$ and $x_7$; $x_5$ representing a value of a fifth clinical parameter, $x_6$ representing a value of a sixth clinical parameter, and $x_7$ representing a value of a seventh clinical parameter.

In some embodiments, $x_p$ can comprise $x_5$, $x_6$, $x_7$ and $x_8$; $x_5$ representing a value of a fifth clinical parameter, $x_6$ representing a value of a sixth clinical parameter, $x_7$ representing a value of a seventh clinical parameter and $x_8$ representing a value of an eighth clinical parameter.

In some embodiments, $x_p$ can comprise $x_5$, $x_6$, $x_7$, $x_8$ and $x_9$; $x_5$ representing a value of a fifth clinical parameter, $x_6$ representing a value of a sixth clinical parameter, $x_7$ representing a value of a seventh clinical parameter, $x_8$ representing a value of an eighth clinical parameter and $x_9$ representing a value of a ninth clinical parameter.

In some embodiments, $x_p$ can comprise $x_5$, $x_6$, $x_7$, $x_8$, $x_9$ and $x_{10}$; $x_5$ representing a value of a fifth clinical parameter, $x_6$ representing a value of a sixth clinical parameter, $x_7$ representing a value of a seventh clinical parameter, $x_8$ representing a value of an eighth clinical parameter, $x_9$ representing a value of a ninth clinical parameter and $x_{10}$ representing a value of a tenth clinical parameter.

In some embodiments, the clinical score of a recipient can be determined using the formula (IIa):

$$p(1|X) = \frac{exp\left(\beta_0 + \sum \beta_i x_i\right)}{1 + exp\left(\beta_0 + \sum \beta_i x_i\right)}$$

wherein:

"$p(1|X)$" represents the clinical score,

- $x = (x_1, x_2, x_3, x_4, ..., x_n)$ a vector of n clinical parameters comprising at least four clinical parameters, wherein

$x_1$ represents a value of a first clinical parameter,
$x_2$ represents a value of a second clinical parameter,
$x_3$ represents a value of a third clinical parameter,
$x_4$ represents a value of a fourth clinical parameter,

$x_n$ represents a value of an additional clinical parameter,
wherein said clinical parameters are chosen independently from the plurality of clinical parameters, and
n represents a number between 0 to 10,

- $\beta_i$ represents the coefficient of the multivariate logistic regression model of each of the plurality clinical parameter, and
- $\beta_0$ represents the intercept coefficient of the model.

The coefficients of the multivariate logistic regression model can be determined using the 'glm' function from the 'stats' package, preferably with version 4.1.3, in R, in conjunction with the boot package, preferably with version 1.3.28.1, for bootstrapping to assess the stability and variability of the estimated coefficients.

In some embodiments, n can consist of 1, 2, 3, 4, 5, 6, 7, 8, 9, or 10.

In some embodiments, $x_n$ can comprise $x_5$; $x_5$ representing a value of a a fifth clinical parameter.

In some embodiments, $x_n$ can comprise $x_5$ and $x_6$; $x_5$ representing a value of a fifth clinical parameter and $x_6$ representing a value of a sixth clinical parameter.

In some embodiments, $x_n$ can comprise $x_5$, $x_6$ and $x_7$; $x_5$ representing a value of a fifth clinical parameter, $x_6$ representing a value of a sixth clinical parameter, and $x_7$ representing a value of a seventh clinical parameter.

In some embodiments, $x_n$ can comprise $x_5$, $x_6$, $x_7$ and $x_8$; $x_5$ representing a value of a fifth clinical parameter, $x_6$ representing a value of a sixth clinical parameter, $x_7$ representing a value of a seventh clinical parameter and $x_8$ representing a value of an eighth clinical parameter.

In some embodiments, $x_n$ can comprise $x_5$, $x_6$, $x_7$, $x_8$ and $x_9$; $x_5$ representing a value of a fifth clinical parameter, x6 representing a value of a sixth clinical parameter, $x_7$ representing a value of a seventh clinical parameter, $x_8$ representing a value of an eighth clinical parameter and $x_9$ representing a value of a ninth clinical parameter.

In some embodiments, $x_n$ can comprise $x_5$, $x_6$, $x_7$, $x_8$, $x_9$ and $x_{10}$; $x_5$ representing a value of a fifth clinical parameter, x6 representing a value of a sixth clinical parameter, $x_7$ representing a value of a seventh clinical parameter, $x_8$ representing a value of an eighth clinical parameter, $x_9$ representing a value of a ninth clinical parameter and $x_{10}$ representing a value of a tenth clinical parameter.

Illustratively, $x_1$ can represent the age of the donor, $x_2$ can represent the Body Mass Index of the donor, $x_3$ can represent the cold ischemia time of the pancreatic transplant, $x_4$ can represent 0 if the recipient received a pretransplant renal replacement treatment or 1 if the recipient did not receive a pretransplant renal replacement treatment, and optionally, a fifth parameter, $x_5$ representing the gender of the donor.

In some particular embodiments, the clinical score can be determined using formula (IIb) :

Formula (IIb) :

$$p(1|X) = \frac{\exp\left(\beta_0 + \beta_{age}x_{age} + \beta_{BMI}x_{BMI} + \beta_{CIT}x_{CIT} + \beta_{preemptive}x_{preemptive}\right)}{1 + \exp\left(\beta_0 + \beta_{age}x_{age} + \beta_{BMI}x_{BMI} + \beta_{CIT}x_{CIT} + \beta_{preemptive}x_{preemptive}\right)}$$

wherein:

- "$p(1|X)$" represents the clinical score,
- $X = (x_{age}, x_{BMI}, x_{CIT}, x_{preemptive})$ a vector of data with

  - $x_{age}$ represents the age of the donor,
  - $x_{BMI}$ represents the Body Mass Index of the donor,
  - $x_{CIT}$ represents the cold ischemia time of the pancreatic transplant,
  - $x_{preemptive}$ represents 0 if the recipient received a pretransplant renal replacement treatment, or 1 if the recipient did not receive a pretransplant renal replacement treatment,

- $\begin{pmatrix} \beta_{age} \\ \beta_{BMI} \\ \beta_{CIT} \\ \beta_{preemptive} \end{pmatrix}$ corresponds to the coefficient of the multivariate logistic regression model of each the clinical parameters, and
- $\beta_0$ represents the intercept coefficient of the multivariate logistic regression model.

In some embodiments, $x_{preemptive}$ represents 0 if the recipient has received a pretransplant dialysis (hemodialyse), or 1 if the recipient did not receive a pretransplant dialysis (hemodialyse).

In some embodiments, the coefficients of the multivariate logistic regression model can be determined using the 'glm' function from the 'stats' package, preferably with version 4.1.3, in R, in conjunction with the boot package, preferably with version 1.3.28.1, for bootstrapping to assess the stability and variability of the estimated coefficients.

In some embodiments, the formula (IIb) can include three or two or one further clinical parameter among the height of the donor, the serum creatinine level of the donor, the gender of the donor, the ethnicity of the donor, the presence or absence of a cerebral vascular accident (CVA) of the donor, the donation after cardiac death (DCD) of the donor and/or the transplantation type.

**Predictive risk score**

The *in vitro* method as described herein comprises a step of determining a **predictive risk score** from the combination of the biological score and the clinical score as previously determined.

This step of determining a predictive risk score can be performed after step (b) and/or step (c) of the method as described herein.

In some embodiments, the predictive risk score can be determined using formula (III) :

(Formula III) :

$$p(1|s_x) = \frac{\exp\left(\beta_0 + \beta_{PFS}s_{x,PFS} + \beta_{clinique}s_{x,clinique}\right)}{1 + \exp\left(\beta_0 + \beta_{PFS}s_{x,PFS} + \beta_{clinique}s_{x,clinique}\right)}$$

wherein:

- "$p(1|s_x)$" represents the predictive risk score for a recipient $x$,
- $s_x = (s_{x,PFS}, s_{x,clinique})$ a vector with

    $s_{X,PFS}$ represents the obtained biological score for the recipient $x$ as described herein,
    $s_{X,clinique}$ represents the obtained clinical score for the recipient $x$ as described herein,

- $\begin{pmatrix} \beta_{PFS} \\ \beta_{clinique} \end{pmatrix}$ corresponds to the coefficients of the multivariable logistic regression model, and
- $\beta_0$ represents the intercept coefficient of the multivariable logistic regression model.

In some embodiments, the coefficients of the logistic regression can be determined using the 'glm' function from the 'stats' package, preferably with version 4.1.3 in R.

The obtained predictive risk score is then compared to a predetermined reference value to assess the prediction of the risk of occurrence of a pancreatic transplant failure and/or a thrombosis.

**Predetermined reference value**

The method as described herein further comprises a step (e) of comparing the predictive risk score, previously obtained in the method, with a predetermined reference value.

This predetermined reference value or threshold can easily be determined by the person skilled in the art based on his or her general knowledge.

In some embodiments, the predetermined reference value can be determined based on a cohort including a plurality of recipients having received a pancreatic transplant from a donor and for whom the occurrence or non-occurrence of a pancreatic transplant failure is known, in particular the occurrence or non-occurrence of an early pancreatic transplant failure is known.

In some embodiments, the predetermined reference value can be a mean predictive risk score obtained by carrying out steps (a) through (d) of the method from a plurality of recipients having received a pancreatic transplant from a donor without having an early pancreatic transplant failure and determining the mean value of each of the predictive risk score obtained in step (d).

In some embodiments, the reference value can be determined based on (i) predictive risk scores determined in recipients without early pancreatic transplant failure and (ii) the predictive risk scores determined in recipients with early pancreatic transplant failure.

This reference value is based on arbitrary units.

The method as described herein can further comprise a step of concluding on the risk of occurrence of the recipient of having a pancreatic transplant failure or thrombosis. Generally, this step is carried out by a clinician.

This step can be performed after step (e) of comparing the predictive risk score at step (d) with a predetermined reference value.

In some embodiments, concluding on the risk of a pancreatic transplant failure for a recipient can refer to :

- determining the risk, or the likelihood of occurrence, of early pancreatic transplantation failure at one month in the recipient after having been grafted or transplanted with the pancreatic transplant; and/or
- determining the benefit/risk balance of establishing a treatment to prevent the occurrence of thrombosis.

In some embodiments, if the predictive risk score is higher than the predetermined reference value, it may be concluded that the recipient is probably at a high risk of occurrence of a pancreas transplant failure. In some embodiments, if the predictive risk score is higher than the predetermined reference value, it may be concluded that the recipient is probably at a high risk of occurrence of having or developing a thrombosis. In some embodiments, if the predictive risk score is largely higher than the predetermined reference value, it may be concluded that the recipient is probably at a very high risk of occurrence of a pancreas transplant failure.

Thus, if the predictive risk score is higher than the predetermined reference value, it can be decided not to proceed with the pancreatic transplant for the given recipient, or it can be decided to provide the pancreatic transplant tested to the given recipient along with a treatment to prevent the occurrence of thrombosis.

In some embodiments, if the predictive risk score is equal or lower than the predetermined reference value, it may be concluded that the recipient is probably at low risk of occurrence of a pancreas transplant failure. In some embodiments, if the predictive risk score is lower than the predetermined reference value, it may be concluded that the recipient is probably at a low risk of occurrence of having or developing a thrombosis. In some embodiments, if the predictive risk score is largely lower than the predetermined reference value, it may be concluded that the recipient is probably at a very lower risk of occurrence of a pancreas transplant failure.

Thus, if the predictive risk score is lower than the predetermined reference value, it can be decided to provide the pancreatic transplant tested to the given recipient, optionally, with a treatment to prevent the occurrence of thrombosis.

In some embodiments, a treatment to prevent the occurrence of thrombosis can be an anticoagulant treatment, an anti-inflammatory drug or any other specific treatment well known by the skilled person in the art to prevent thrombosis after pancreatic transplantation.

It is understood that these conclusions can vary slightly, as it is ultimately the clinician's responsibility to interpret the results of the comparison between the predictive risk score and the predetermined reference value, and to establish a conclusion on the risk of occurrence and/or a preventive treatment.

The present disclosure also relates to an *in vitro* method for predicting the risk of occurrence of a pancreatic transplant failure in a recipient intended to receive a pancreatic transplant, comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) comparing the biological score obtained at step b) with a predetermined reference biological score determined in at least one reference recipient, wherein the reference recipient did not receive a pancreatic transplant; and, optionally,
d) concluding that the recipient is at high risk of having a pancreatic transplant failure if the biological score obtained at step b) is lower than the predetermined reference biological score.

Indeed, the analysis of the values of the three protein biomarkers clearly demonstrated two distinguished patient clusters corresponding to the recipients with early pancreatic transplant failure and the control recipients, respectively. Recipient with early pancreatic transplant failure showed increased MMP-9 values associated with low VEGF and Angiopoietin 2 values, whereas control recipients showed low MMP-9 values and increased VEGF and Angiopoietin 2 values. To represent the combination of these three biomarkers, a biological score was calculated.

The present disclosure also relates to the uses of a **biological score** for predicting the risk of a pancreatic transplant failure in a recipient intended to receive a pancreatic transplant, wherein the **biological score** is determined from the level of at least one protein biomarker selected from matrix metalloproteinase 9 (MMP-9), Vascular endothelial growth factor (VEGF) and Angiopoietin-2 (ANGPT2), measured in a biological sample previously obtained from the recipient.

In some embodiments, the predetermined reference biological score can be determined from the level of the protein biomarkers from a plurality of reference recipients.

In some embodiments, the methods can comprise a step d) of concluding the risk of occurrence of having a pancreatic transplant failure for the recipient based on the comparison of the biological score obtained at step b) with a predetermined reference biological score

In some embodiments, the methods can comprise a step d) of concluding that the recipient is at high risk of having a pancreatic transplant failure if the biological score obtained at step b) is lower than the predetermined reference biological score, or concluding that the recipient is at low risk of having a pancreatic transplant failure if the biological score obtained at step b) is higher than the predetermined reference biological score.

It is understood that the features defined in the present disclosure for the methods and uses wherein it is determined a predictive risk score apply to the above methods and uses implementing solely the biological score.

## Method for preventing a thrombosis

The present disclosure also relates to a method for preventing a thrombosis in a recipient intended to receive a pancreatic transplant, comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) determining a **clinical score** from a plurality of clinical parameters from the donor and the recipient, these parameters being associated with a pancreatic transplant failure,
d) determining a **predictive risk score** from the combination of the biological score determined in step (b) and the clinical score determined in step (c),
e) comparing the predictive risk score obtained at step (d) with a predetermined reference value, and, optionally,
f) concluding that the recipient is at high risk of having a thrombosis if the predictive risk score is higher than the predetermined reference value.

As previously stated, thrombosis of the pancreatic transplant is the primary cause of graft/transplant failure in the first year. Prior knowledge of the individualized risk of pancreatic transplant failure allows for the implementation of specific treatments to prevent thrombotic complications. Currently, these treatments are not employed on a routine basis due to the associated risk of haemorrhage and/or infection in the immediate post transplantation period. In recipients identified as being at high risk of pancreatic transplant failure or transplant thrombosis, such treatment could be considered without exposing all patients at low risk of thrombosis to the risk of haemorrhage and/or infection.

In some embodiments, if the predictive risk score is higher than the predetermined reference value, it may be concluded that the recipient is probably at a high risk of occurrence of having or developing a thrombosis. In some embodiments, if the predictive risk score is largely higher than the predetermined reference value, it may be concluded that the recipient is probably at a very high risk of occurrence of having or developing a thrombosis.

In some embodiments, if the predictive risk score is lower than the predetermined reference value, it may be concluded that the recipient is probably at a low risk of occurrence of having or developing a thrombosis. In some embodiments, if the predictive risk score is largely lower than the predetermined reference value, it may be concluded that the recipient is probably at a very low risk of occurrence of having or developing a thrombosis.

In some embodiments, the method can further comprise an additional step wherein if it is concluded that the recipient is at high risk of having a thrombosis, it can be administered to the recipient a treatment to prevent the occurrence of thrombosis. This treatment can be administered before the transplant surgery and/or after the transplant surgery.

In some embodiments, a treatment to prevent the occurrence of thrombosis can be an anticoagulant treatment, an anti-inflammatory drug or any other specific treatment well known by the skilled person in the art to prevent thrombosis after pancreatic transplantation.

It is understood that these conclusions can vary slightly, as it is ultimately the clinician's responsibility to interpret the results of the comparison between the predictive risk score and the predetermined reference value, and to establish a conclusion on the risk of occurrence and/or a preventive treatment.

The present disclosure also relates to a method for preventing a thrombosis in a recipient intended to receive a pancreatic transplant, comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) comparing the biological score obtained at step b) with a predetermined reference biological score determined in at least one reference recipient, wherein the reference recipient did not receive a pancreatic transplant; and

d) concluding that the recipient is at high risk of having a pancreatic transplant failure if the biological score obtained at step b) is lower than the predetermined reference biological score.

In some embodiments, if the biological score is higher than the predetermined reference biological value, it may be concluded that the recipient is probably at low risk of occurrence of having or developing a thrombosis. In some embodiments, if the predictive risk score is largely higher than the predetermined reference value, it may be concluded that the recipient is probably at a very low risk of occurrence of having or developing a thrombosis.

In some embodiments, if the predictive risk score is lower than the predetermined reference value, it may be concluded that the recipient is probably at high risk of occurrence of having or developing a thrombosis. In some embodiments, if the predictive risk score is largely lower than the predetermined reference value, it may be concluded that the recipient is probably at a very high risk of occurrence of having or developing a thrombosis.

In some embodiments, the method can further comprise an additional step wherein if it is concluded that the recipient is at high risk of having a thrombosis, it can be administered to the recipient a treatment to prevent the occurrence of thrombosis. This treatment can be administered before the transplant surgery and/or after the transplant surgery.

In some embodiments, a treatment to prevent the occurrence of thrombosis can be an anticoagulant treatment, an anti-inflammatory drug or any other specific treatment well known by the skilled person in the art to prevent thrombosis after pancreatic transplantation.

It is understood that these conclusions can vary slightly, as it is ultimately the clinician's responsibility to interpret the results of the comparison between the biological score and the predetermined reference biological value, and to establish a conclusion on the risk of occurrence and/or a preventive treatment.

It is understood that the features defined in the present disclosure for the methods and uses as described herein apply for the above methods for preventing a thrombosis.

## Computer-implemented methods

The methods according to the present disclosure can be computer-implemented methods.

The present invention may also relate to a computer system for predicting the risk, or the likelihood, of occurrence of a pancreatic transplant failure or thrombosis in a recipient intended to receive a pancreatic transplant. The present invention also related to a computer-implemented method for predicting the risk, or the likelihood, of occurrence of a pancreatic transplant failure or thrombosis in a recipient intended to receive a pancreatic transplant.

In some embodiments, the determination of the biological score, the clinical score and/or the predictive risk score can be performed by means of computer programs automatically on any electronic system comprising a processor, especially a computer.

In some embodiments, the methods according to the present disclosure are advantageously performed by means of computer programs automatically on any electronic system comprising a processor, especially a computer.

A computer program may include a sequence of instructions, executable in the digital processing device's CPU, written to perform a specified task. Computer readable instructions may be implemented as program modules, such as functions, objects, Application Programming Interfaces (APIs), data structures, and the like, that perform particular tasks or implement particular abstract data types. A computer program may be written in various versions of various languages.

In some embodiments, a computer program includes, in part or in whole, one or more web applications, one or more mobile applications, one or more standalone applications, one or more web browser plug-ins, extensions, add-ins, or add-ons, or combinations thereof.

In another aspect, the present disclosure relates to a computer program product comprising a support and stored on this support instructions that can be read by a processor, these instructions being configured to assess a predictive risk score, from the combination of a biological score as described herein and a clinical score as described herein according to the present disclosure, and, optionally, to determine that a recipient is at high risk of having a pancreatic transplant failure if the predictive risk score is higher than a predetermined reference value or concluding that the recipient is at low risk of having a pancreatic transplant failure if the predictive risk score is lower than the predetermined reference value according to the present disclosure.

In some embodiments, the computer program product can only assess or determine the predictive risk score as described herein.

In some embodiments, the step of concluding that the recipient is at high risk or at low risk of having a pancreatic transplant failure is determined by an individual.

In some embodiments, the instructions of the computer program product can only assess or determine the predictive risk score as described herein.

In another aspect, the present disclosure relates to a computer program product comprising a support and stored on this support instructions that can be read by a processor, these instructions being configured to assess a biological score as described herein, and, optionally, to determine that a recipient is at high risk of having a pancreatic transplant failure if the biological score is lower than a predetermined reference biological score.

The present disclosure also relates to a computer readable storage medium comprising the computer program product(s) as described herein. Examples of computer-readable storage medium include, but are not limited to, an integrated circuit, a hard disk, a magnetic tape (including floppy disk and zip diskette), an optical disc (including Blu-ray, compact disc and digital versatile disc), a flash memory (including memory card and USB flash drive) a random-access memory (RAM) (including dynamic and static RAM), a read-only memory (ROM) or a cache.

The features defined in the present disclosure for the methods apply to the computer program products.

## EXAMPLES

### I. Determination of a clinical score for pancreatic graft failure based on clinical parameters

From the Agence de Biomédecine national registry, all pancreatic transplants performed between 2000 and 2022 were analyzed (n = 1665). A multivariate logistic regression model was performed to determine the variables significantly associated with pancreatic graft failure in the 30 days post-transplant. A stepwise regression was applied to select the variables allowing the construction of a predictive score for pancreatic graft failure. The bootstrap method was applied to avoid overfitting.

Four clinical parameters were determined as significantly associated with pancreatic graft failure at 30 days post-transplant: the donor age (OR = 1.022 [1.006; 1.038] ;p = 0.009), the donor BMI (OR = 1.066 [1.011; 1.125] ; p = 0.02) and the preemptive status of the recipient (presence or absence of pre-transplant hemodialysis) (OR = 2.769 [1.982; 3.916] ; p <0.0001) and the cold ischemia time of the pancreatic transplant (Ref 1-7 ; 8-14: OR = 1.044 [0.629; 1.780] ; p=0.87 ; 15-22: OR = 1.202 [0.590; 2.459] ; p=0.61). The combination of these four parameters gave an AUC of 0.67.

From these clinical parameters, a clinical score has been determined using the formula (II):

$$p(1|X) = \frac{\exp(\beta_0 + \beta_{age}X_{age} + \beta_{BMI}X_{BMI} + \beta_{CIT}X_{CIT} + \beta_{preemptive}X_{preemptive})}{1 + \exp(\beta_0 + \beta_{age}X_{age} + \beta_{BMI}X_{BMI} + \beta_{CIT}X_{CIT} + \beta_{preemptive}X_{preemptive})}$$

wherein:

"$p(1|X)$" represents the probability of allograft failure at day 30 post-transplantation (clinical score),
$X = (X_{age}, X_{BMI}, X_{CIT}, X_{preemptive})$ a matrix of data with:

- $X_{age}$ a vector with values of the age of the donors,
- $X_{BMI}$ a vector with values of the body mass index of the donors,
- $X_{CIT}$ a vector with values of the cold ischemia time of the pancreatic transplants,
- $X_{preemptive}$ a vector with value 0 if the recipient received a pretransplant hemodialysis, or 1 if the recipient did not receive a pretransplant hemodialysis,

$$\beta = \begin{pmatrix} \beta_{age} \\ \beta_{BMI} \\ \beta_{CIT} \\ \beta_{preemptive} \end{pmatrix}$$ corresponds to the coefficients of the multivariate logistic regression model we want to calculate, and
$\beta_0$ refers to the intercept coefficient of the model, we want to calculate.

Packages R used: glm (library stats version 4.1.3) and boot (library boot version 1.3.28.1)

### II. Determination of a biological score from biomarkers in the recipient associated with graft failure at 30 days post-transplant.

From the Nantes DIVAT biocollection, 29 patients with a failed pancreas transplant within the first month and with a serum sample available at D0 (pre-transplant) were selected ("cases"). A group of 29 control patients, matched according to donor failure risk criteria defined by the previously constructed clinical score (donor age, donor BMI), was selected as the "control" group. All pre-transplant sera from these patients were analyzed using LEGENDPLEX (BioLegend), a technique enabling simultaneous quantification of several analytes (n = 14 max) in a single sample, with results measured by flow cytometry. Using several pre-established kits, this technique enabled simultaneous analysis of some sixty inflammatory and haemostatic serum biomarkers present at D0 of transplantation (pre-transplantation) in patients in the "case" and "control" groups. From the combination of biomarkers expressed differently in the groups, a biological prediction score was calculated.

Of the 59 biomarkers analysed, 3 were differentially expressed in the case group compared with the control group.

In patients with graft failure due to thrombosis within the first 30 days post transplantation, the level of MMP9 in serum sample at D0 is higher (4848 vs. 2584 pg/ml, p = 0.03), while the levels of VEGF and Angiopoietin II are lower (92.4 vs. 133.8 pg/ml, p = 0.05 and 2864 vs. 4068 pg/ml p = 0.09 respectively). A principal component analysis was performed on the values of these three protein biomarkers. This enabled the inventors to effectively distinguish two patient clusters corresponding to "case" and "control" patients. Indeed, "case" patients showed increased MMP-9 values associated with low VEGF and Angiopoietin 2 values, whereas "control" patients showed low MMP-9 values and increased VEGF and Angiopoietin 2 values.

To represent the combination of these three markers, a biological score was calculated with formula (I):

[log10 (level of VEGF)] + [log10 (level of Angio)] - [log10 (level of MMP-9)].               Formula (I)

Biological scores (PFS) were significantly lower in the thrombosis failure group (cases) than in the control group: 1.77 vs. 2.39, p = 0.008 (Figure 2A). The value of the biological score, a combination of VEGF, Angiopoietin II and MMP9 efficiently discriminate recipients who are at high risk of early pancreas transplant failure. Evaluation of predictive capacity based on this biological score alone revealed an AUC of 0.73 (Figure 2B).

The establishment of a biological score based on the direct measurement of three analytes makes it easier to implement in clinical routine than using a principal component analysis.

## III. Determination of the predictive risk score by combining the clinical score and the biological score to predict the risk of pancreatic transplant failure at day 30 post-transplant

The predictive capacity of the clinical score, the biological score, and their combination, i.e., the predictive risk score, of the 58 patients, was evaluated and compared with the Gold Standard used in pancreatic transplantation, the North American PDRI score (Pancreas Donor Risk Index score) (Axelrod, et al. Systematic Evaluation of Pancreas Allograft Quality, Outcomes and Geographic Variation in Utilization. Am. J. Transplant. 10, 837-845 (2010*)) (Table 1)*.

In this group of patients, the predictive capacity of the **PDRI** was assessed with an AUC of 0.49 (Sensitivity 68%, Specificity 41%, PPV 54%, NPV 57%).

The predictive capacity of the **clinical score alone** was evaluated with a AUC of 0.62 (Sensitivity 72%, Specificity 41%, PPV 55%, NPV 60%). This lower AUC value compared with the clinical score established on the whole cohort (0.62 vs. 0.67) can be explained in part by the selection of "case/control" patients. Indeed, donor age and donor BMI are two parameters on which the selection of "controls" is made in relation to "case" patients. However, these two parameters are included in the clinical score.

The predictive capacity of the **biological score alone** showed an AUC of 0.73 (Sensitivity 69%, Specificity 72%, PPV 71%, NPV 70%).

The predictive capacity of the **predictive risk score**, combining the clinical score and biological score values, showed an AUC of 0.74 (Sensitivity 72%, Specificity 69%, PPV 70%, NPV 71%). As before, the similarity between the predictive capacity results of the biological score and of the predictive risk score is explained by the selection of patients, who were voluntarily adjusted for donor risk parameters, in order to best determine the intrinsic risk relative to the recipient.

The predictive risk score was determined using a formula (III) :

$$p(1|S_X) = \frac{\exp\left(\beta_0 + \beta_{PFS}S_{X,PFS} + \beta_{clinique}S_{X,clinique}\right)}{1 + \exp\left(\beta_0 + \beta_{PFS}S_{X,PFS} + \beta_{clinique}S_{X,clinique}\right)}$$

wherein:

"$p(1|S_X)$" represents the probability of allograft failure at day 30 post-transplantation with the association of PFS and the clinical score calculated with X data (predictive risk score),

$S_X = (S_{X,PFS}, S_{X,clinique})$ a matrix with:

- $S_{X,PFS}$ vector of the previously obtained biological score calculated for the recipients in $X$,
- $S_{X,clinique}$ vector of the previously obtained clinical score calculated for the recipients in $X$,

$$\beta_{score} = \begin{pmatrix} \beta_{PFS} \\ \beta_{clinique} \end{pmatrix}$$ corresponds to the coefficients of the multivariable logistic regression of the biological and clinical scores,

$\beta_0$ refers to the intercept coefficient of the multivariable logistic regression model.

Packages R used: glm (library stats version 4.1.3).

In contrast to the PDRI and the P-PASS (European score), the predictive risk score was constructed by selecting the clinical variables of interest using biostatistical selection (stepwise logistic regression) rather than selection based on the risk factors described in the literature.

Most importantly, the predictive risk score combines clinical parameters with the value of three serum protein biomarkers (MMP-9, VEGF, and ANGPT2) present in the recipient at day 0 of transplantation in order to enhance predictive capacity.

The combination of clinical parameters and protein biomarkers produced an area under the curve (AUC) that was significantly greater than that of the PDRI score for pancreatic graft survival at day 30 post-transplantation: AUC = 0.74 vs 0.49. This difference in prediction was confirmed when assessing pancreatic graft survival at one-year post-transplant (AUC = 0.73 vs 0.46).

Table 1: Comparison of the predictive capacity of the predictive risk score, biological score and clinical score to the PDRI.

|  | Specificity | Sensitivity | PPV | NPV | Accuracy | F1 score | AUC |
|---|---|---|---|---|---|---|---|
| PDRI | 0.41 | 0.68 | 0.54 | 0.57 | 0.55 | 0.60 | 0.49 |
| Clinical score | 0.41 | 0.72 | 0.55 | 0.60 | 0.56 | 0.62 | 0.62 |
| Biological score | 0.72 | 0.72 | 0.72 | 0.72 | 0.72 | 0.72 | 0.73 |
| Predicting risk score | 0.69 | 0.76 | 0.71 | 0.71 | 0.72 | 0.73 | 0.74 |

**IV. Determination and evaluation of the predetermined reference score (threshold) to compare the predictive risk score of the recipient and concluding on the risk of early transplant failure**

The determination of the predetermined reference score (threshold) can be performed in a more complete cohort of individuals having received a pancreatic transplant from a donor and for whom the occurrence or non-occurrence of an early pancreatic transplant failure is known, according to the above-described steps and by carrying out the formula (I), (II) and (II) to determine the predictive risk score.

The Youden's J index can be used: J_index = sensitivity + specificity - 1 with "sensitivity" = TP/(TP+FN) and "specificity" = TN/(TN+FP), wherein "TP" corresponds to the number of True Positives; "FN" corresponds to the number of False Negatives; "TN" corresponds to the number of True Negatives; and "FP" corresponds to the number of False Positives.

The more the J index is close to 1, the better are the results. This index allows to have less false positive values.

Another measure that can be used is the Matthew's correlation coefficient (MCC). The MCC can be calculated directly from the confusion matrix using the formula:

$$\text{MCC} = (\text{TN} \times \text{TP} - \text{FN} \times \text{FP}) / \sqrt{((\text{TP}+\text{FP})(\text{TP}+\text{FN})(\text{TN}+\text{FP})(\text{TN}+\text{FN}))}$$

wherein "TP" corresponds to the number of True Positives; "FN" corresponds to the number of False Negatives; "TN" corresponds to the number of True Negatives; and "FP" corresponds to the number of False Positives. If any of the four sums in the denominator is zero, the denominator can be arbitrarily set to one; this results in a Matthews correlation coefficient of zero, which can be shown to be the correct limiting value.

Finally, the F1 score will be evaluated to determine the predetermined reference score for predicting pancreas transplant failure.

**REFERENCES**

Axelrod, D. A., Sung, R. S., Meyer, K. H., Wolfe, R. A. & Kaufman, D. B. Systematic Evaluation of Pancreas Allograft Quality, Outcomes and Geographic Variation in Utilization. Am. J. Transplant. 10, 837-845 (2010)
Finger, E. B. et al. A Composite Risk Model for Predicting Technical Failure in Pancreas Transplantation. Am. J. Transplant. 13, 1840-1849 (2013).

**Claims**

1. An *in vitro* method for predicting the risk of occurrence of a pancreatic transplant failure in a recipient intended to receive a pancreatic transplant, wherein the pancreatic transplant originates from a donor, the method comprising at least the steps of:

   a) providing a biological sample previously obtained from the recipient,
   b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
   c) determining a **clinical score** from a plurality of clinical parameters from the donor and the recipient, these parameters being associated with a pancreatic transplant failure,
   d) determining a **predictive risk score** from the combination of the biological score determined in step (b) and the clinical score determined in step (c),
   e) comparing the predictive risk score obtained at step (d) with a predetermined reference value, and
   f) concluding that the recipient is at high risk of having a pancreatic transplant failure if the predictive risk score is higher than the predetermined reference value or concluding that the recipient is at low risk of having a pancreatic transplant failure if the predictive risk score is lower than the predetermined reference value.

2. The *in vitro* method according to claim 1, wherein the biological sample is chosen from whole blood, plasma and serum, in particular le biological sample is serum.

3. The *in vitro* method according to claim 1 or 2, wherein the biological score is determined at step (b) using the formula (I):

   *[log₁₀ (level of VEGF)] + [log₁₀ (level of ANGPT2)] - [log₁₀ (level of MMP- 9)].*                    Formula (I) :

4. The *in vitro* method according to any one of claims 1 to 3, wherein the level of the at least one biomarker at step (b) is measured with Flow Cytometry, enzyme immunoassay or Mass Spectrometry.

5. The *in vitro* method according to any one of claims 1 to 4, wherein the plurality of clinical parameters includes the age of the donor, the Body Mass Index (BMI) of the donor, the cold ischemia time of the pancreatic transplant and the presence or absence of renal replacement treatment from the recipient.

6. The *in vitro* method according to any one of claims 1 to 5, wherein the clinical score is determined using a formula (II) :

   Formula (II) :

$$p(1|x) = \frac{\exp\left(\beta_0 + \sum_{i=1}^{p} \beta_i x_i\right)}{1 + \exp\left(\beta_0 + \sum_{i=1}^{p} \beta_i x_i\right)}$$

   wherein:

   - "$p(1|x)$" represents the clinical score,
   - $x = (x_1, ..., x_i, ... , x_p)$ a vector of size p with the values of the clinical parameters,
   - $\beta = \begin{pmatrix} \beta_1 \\ ... \\ \beta_p \end{pmatrix}$ a vector of coefficients determined with the multivariable logistic regression, and
   - $\beta_0$ refers to the intercept coefficient of the multivariate logistic regression model.

7. The *in vitro* method according to any one of the preceding claims, wherein the predictive risk score is determined using a formula (III) :

(Formula III) :

$$p(1|s_x) = \frac{\exp\left(\beta_0 + \beta_{PFS}s_{x,PFS} + \beta_{clinique}s_{x,clinique}\right)}{1 + \exp\left(\beta_0 + \beta_{PFS}s_{x,PFS} + \beta_{clinique}s_{x,clinique}\right)}$$

wherein:

- "$p(1|s_x)$" represents the predictive risk score,
- $s_x = (s_{x,PFS}, s_{x,clinique})$ a vector with

   $s_{X,PFS}$ represents the obtained biological score,
   $s_{X,clinique}$ represents the obtained clinical score,

- $\begin{pmatrix} \beta_{PFS} \\ \beta_{clinique} \end{pmatrix}$ corresponds to the coefficients of the multivariable logistic regression model, and
- $\beta_0$ represents the intercept coefficient of the multivariable logistic regression model.

8. The *in vitro* method according to any one of the preceding claims, wherein the predetermined reference value consists of a value determined from a biological sample of a plurality of recipients having received a pancreatic transplant and for whom the occurrence or non-occurrence of a pancreatic transplant failure is known, in particular the occurrence or non-occurrence of a pancreatic transplant failure is known at about one-month post-transplantation.

9. The *in vitro* method according to any one of the preceding claims, wherein the risk of a pancreatic transplant failure consists of :

   - determining the risk, or the likelihood of occurrence, of early pancreatic transplantation failure at one month or more in the recipient after having been transplanted with the pancreatic transplant; and/or
   - determining the benefit/risk balance of establishing a to prevent the occurrence of thrombosis.

10. The *in vitro* method according to any one of the preceding claims, wherein the recipient has a diabetes, a chronic pancreatitis, a pancreatic cancer and/or a pancreatic insufficiency.

11. The *in vitro* method according to any one of the preceding claims, wherein the recipient and the donor consist of mammals, in particular humans.

12. An *in vitro* method for predicting the risk of a pancreatic transplant failure in a recipient intended to receive a pancreatic transplant, comprising at least the steps of:

   a) providing a biological sample previously obtained from the recipient,
   b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
   c) comparing the biological score obtained at step b) with a predetermined reference biological score determined in at least one reference recipient, wherein the reference recipient did not receive a pancreatic transplant; and
   d) concluding that the recipient is at high risk of having a pancreatic transplant failure if the biological score obtained at step b) is lower than the predetermined reference biological score.

13. Use of a **predictive risk score** for predicting the risk of a pancreatic transplant failure in a recipient intended to receive a pancreatic transplant, the predictive risk score being determined from the combination of a biological score and a clinical score; wherein the **biological score** is determined from the level of at least one protein biomarker selected from matrix metalloproteinase 9 (MMP-9), Vascular endothelial growth factor (VEGF) and Angiopoietin-2 (ANGPT2), measured in a biological sample previously obtained from the recipient, and the **clinical score** is determined from a plurality of clinical parameters from the donor and the recipient, the clinical parameters being associated with a pancreatic transplant failure.

**14.** Use of a **biological score** for predicting the risk of a pancreatic transplant failure in a recipient intended to receive a pancreatic transplant, wherein the **biological score** is determined from the level of at least one protein biomarker selected from matrix metalloproteinase 9 (MMP-9), Vascular endothelial growth factor (VEGF) and Angiopoietin-2 (ANGPT2), measured in a biological sample previously obtained from the recipient.

**15.** A method for preventing a thrombosis in a recipient intended to receive a pancreatic transplant, comprising at least the steps of:

a) providing a biological sample previously obtained from the recipient,
b) determining a **biological score** from the level of at least one protein biomarker selected from matrix metalloproteinase 9 (MMP-9), Vascular endothelial growth factor (VEGF) and Angiopoietin-2 (ANGPT2), measured in the biological sample,
c) determining a **clinical score** from a plurality of clinical parameters from the donor and the recipient, these parameters being associated with a pancreatic transplant failure,
d) determining a **predictive risk score** from the combination of the biological score determined in step (b) and the clinical score determined in step (c),
e) comparing the predictive risk score obtained at step (d) with a predetermined reference value, and
f) concluding that the recipient is at high risk of having a thrombosis if the predictive risk score is higher than the predetermined reference value.

**FIGURE 1**

**FIGURE 2A**

**FIGURE 2B**

Europäisches
Patentamt

European
Patent Office

Office européen
des brevets

# EUROPEAN SEARCH REPORT

**Application Number**

EP 24 30 5967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JAHN N. ET AL.: "Correlation of different serum biomarkers with prediction of early pancreatic graft dysfunction following simultaneous pancreas and kidney transplantation", J. CLIN. MED., vol. 11, no. 9, 2563, 3 May 2022 (2022-05-03), pages 1-21, XP093230403, * abstract * * figure 1; tables 1,3,5,6,7 * * page 13 - page 16 * ----- | 1-15 | INV. G01N33/68 |
| Y | MASSET C. ET AL.: "The role of donor hypertension and angiotensin II in the occurrence of early pancreas allograft thrombosis", FRONT. IMMUNOL., vol. 15, 1359381, 17 May 2024 (2024-05-17), pages 1-10, XP093230297, * the whole document * * page 5 * * tables 2, S1, S2 * ----- | 1-15 | |
| Y | FELLMER P.T. ET AL.: "Influence of donor- and recipient-specific factors on the postoperative course after combined pancreas-kidney transplantation", LANGENBECKS ARCH. SURG., vol. 395, no. 1, 3 September 2009 (2009-09-03), pages 19-25, XP093230303, * the whole document * * page 24 - page 25 * ----- -/-- | 1-15 | TECHNICAL FIELDS SEARCHED (IPC) G01N |

The present search report has been drawn up for all claims

| Place of search | Date of completion of the search | Examiner |
|---|---|---|
| Munich | 3 December 2024 | Giry, Murielle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

## EUROPEAN SEARCH REPORT

**Application Number**

EP 24 30 5967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/199781 A1 (BRISCOE D.M. [US] ET AL.) 17 July 2014 (2014-07-17) <br> * the whole document * <br> * paragraphs [0005], [0095] - [0114] * <br> * tables 1-4 * <br> * claims 1-70 * | 1-15 | |
| Y | WO 2024/068521 A1 (FUNDACIÓN PARA LA FORMAC. E INVESTIG. SANITARIA DE LA REGIÓN DE MURCIA) 4 April 2024 (2024-04-04) <br> * the whole document * <br> * tables 3,4,8,9 * <br> * claims 1-10 * | 1-15 | |
| A,D | FINGER E.B. ET AL.: "A composite risk model for predicting technical failure in pancreas transplantation", AM. J. TRANSPLANTATION, vol. 13, no. 7, 24 May 2013 (2013-05-24), pages 1840-1849, XP072347811, * the whole document * | 1-15 | |
| A,D | AXELROD D.A. ET AL.: "Systematic evaluation of pancreas allograft quality, outcomes and geographic variation in utilization", AM. J. TRANSPLANTATION, vol. 10, no. 4, February 2010 (2010-02), pages 837-845, XP072345505, * the whole document * | 1-15 | **TECHNICAL FIELDS SEARCHED (IPC)** |

The present search report has been drawn up for all claims

| Place of search | Date of completion of the search | Examiner |
|---|---|---|
| Munich | 3 December 2024 | Giry, Murielle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

page 2 of 2

# EP 4 667 938 A1

## ANNEX TO THE EUROPEAN SEARCH REPORT
## ON EUROPEAN PATENT APPLICATION NO.

EP 24 30 5967

03-12-2024

| Patent document cited in search report | Publication date | Patent family member(s) | Publication date |
|---|---|---|---|
| US 2014199781 A1 | 17-07-2014 | US 2014199781 A1<br>WO 2012122374 A2 | 17-07-2014<br>13-09-2012 |
| WO 2024068521 A1 | 04-04-2024 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AXELROD et al.** *Am. J. Transplant.*, 2010, vol. 10, 837-845
- **FINGER et al.** *Am. J. Transplant.*, 2013, vol. 13, 1840-1849
- **AXELROD et al.** Systematic Evaluation of Pancreas Allograft Quality, Outcomes and Geographic Variation in Utilization.. *Am. J. Transplant.*, 2010, vol. 10, 837-845

- **AXELROD, D. A.** ; **SUNG, R. S.** ; **MEYER, K. H.** ; **WOLFE, R. A.** ; **KAUFMAN, D. B.** Systematic Evaluation of Pancreas Allograft Quality, Outcomes and Geographic Variation in Utilization.. *Am. J. Transplant*, 2010, vol. 10, 837-845
- **FINGER, E. B. et al.** A Composite Risk Model for Predicting Technical Failure in Pancreas Transplantation.. *Am. J. Transplant.*, 2013, vol. 13, 1840-1849